(19) Europäisches Patentamt
European Patent Office
Office européen des brevets

(11) **EP 2 876 081 A1**

(12) **EUROPÄISCHE PATENTANMELDUNG**

(43) Veröffentlichungstag:
**27.05.2015 Patentblatt 2015/22**

(21) Anmeldenummer: **13194431.6**

(22) Anmeldetag: **26.11.2013**

(51) Int Cl.:
***C01B 17/00*** *(2006.01)*
***C07C 211/62*** *(2006.01)*
***C07C 211/63*** *(2006.01)*
***C07C 211/64*** *(2006.01)*
***C07C 279/04*** *(2006.01)*
***C07C 321/12*** *(2006.01)*
***C07C 381/00*** *(2006.01)*
***C07C 381/12*** *(2006.01)*
***C07C 391/00*** *(2006.01)*
***C07C 395/00*** *(2006.01)*
***C07D 213/20*** *(2006.01)*
***C07D 231/12*** *(2006.01)*
***C07D 233/56*** *(2006.01)*
***C07F 9/54*** *(2006.01)*
***H01B 1/12*** *(2006.01)*
*C01B 19/00 (2006.01)*
*H01G 9/20 (2006.01)*
*H01L 31/042 (2014.01)*
*H01M 6/16 (2006.01)*
*H01M 10/0569 (2010.01)*

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(71) Anmelder: **PHILIPPS-UNIVERSITÄT MARBURG**
**35037 Marburg (DE)**

(72) Erfinder:
- **Sundermeyer, Jörg**
  **35041 Marburg (DE)**
- **Finger, Lars Hendrik**
  **24884 Selk (DE)**

(74) Vertreter: **Stumpf, Peter**
**c/o TransMIT GmbH**
**Kerkrader Strasse 3**
**35394 Gießen (DE)**

(54) **Ionische Flüssigkeiten mit einem Chalkogenid-Anion**

(57) Die Erfindung betrifft die Herstellung einer ionischen Flüssigkeit mit einem Chalkogenid-Anion aus einer ionischen Flüssigkeit mit einem Carbonat-Anion durch Anionenaustausch.

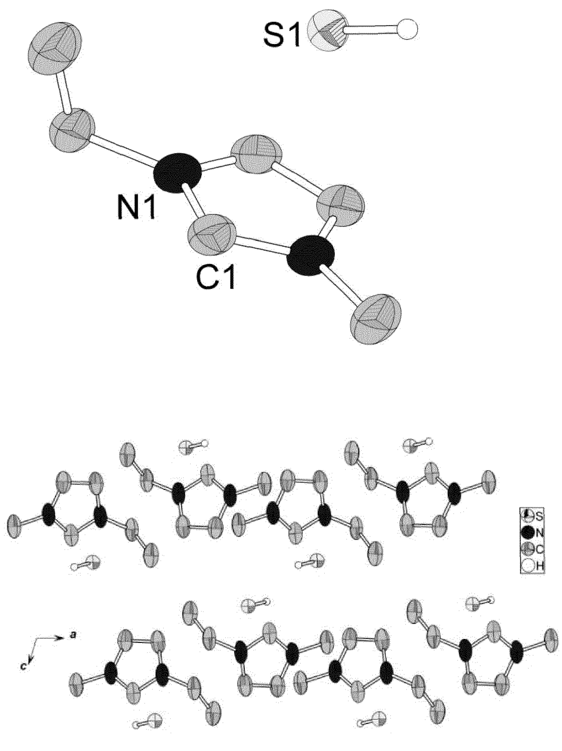

Fig. 1

EP 2 876 081 A1

## Beschreibung

Die vorliegende Erfindung betrifft ionische Flüssigkeiten mit einem Chalkogenid-Anion. Chalkogenid umfasst dabei Schwefel, Selen und Tellur. Die Herstellung erfolgt bevorzugt durch Anionenaustausch des Organocarbonat-Anions mit dem Chalkogenid-Anion.

## [Stand der Technik]

In der WO 2011/144697 wurden ionische Flüssigkeiten mit einem Hydrogensulfid-Anion offenbart, die durch Umsetzung einer ionischen Flüssigkeit mit Alkalisulfidhydraten hergestellt wurden. Durch Erhitzen im Feinvakuum sollen ionische Flüssigkeiten mit einem Sulfid-Dianion gewonnen werden können. Nachteilig ist hierbei, dass die Herstellung von Selenid und Tellurid nicht praktisch offenbart ist und unter wässrigen Bedingungen in organischen Lösungsmitteln wie Aceton gearbeitet wird. Es ist bekannt, dass Aceton unter basischen Bedingungen in Gegenwart von Sulfid zu schwer abtrennbaren Aceton-Kondensationsprodukten führt. Weiterhin sind die durch diese Art des Anionaustausches gewonnenen ionischen Flüssigkeiten zwangläufig mit Verunreinigungen durch Metallkationen und Halogenidanionen verunreinigt. Aufgrund organischer Verunreinigungen, Restmengen an Wasser und Fremdkationen sowie Fremdanionen eignen sich die nach Stand der Technik gewonnenen Sulfid-ionische Flüssigkeiten nicht für Anwendungen in der Batterietechnologie, wo es auf höchste Reinheiten der Elektrolyte ankommt. Wünschenswert wäre eine Technologie, die wasserfrei arbeitet, die mit leicht flüchtigen organischen Lösemitteln arbeitet, keine organischen Lösemittel, die zu Kondensation neigen, und die im Produktiosprozess keine Fremdionen und Salzlasten abtrennen muss.
In der CN 102097213 B wurde nach einem Anionenaustausch von Chlorid gegen Hydroxid ein zweiwertiges Sulfid durch zweifache Deprotonierung von Schwefelwasserstoff hergestellt. Nachteilig ist die mehrstufige Synthese und erneut die Arbeit unter wässrigen Bedingungen. Bei der aufwändigen Herstellung des als Startmaterial verwendeten wässrigen Hydroxidsalzes ist dessen Kontamination durch Wasser, organische Komponenten von Ionenaustauschern sowie durch Fremdionen nicht oder nur sehr schwer auszuschließen. Zudem ist Tellurwasserstoff so instabil, dass es nicht in Gasdruckflaschen erhältlich ist. Daher ist dieses Verfahren möglicherweise nur für Schwefelwasserstoff geeignet.
In der WO 98/11619 wurden ionische Flüssigkeiten mit einem Sulfid-Anion als Elektrolyte eingesetzt. Allerdings wurde die Herstellung nicht beschrieben.

Ein Anionenaustausch mit Wasserstoffselenid und Wasserstofftellurid zu entsprechenden ionischen Flüssigkeiten ist deutlich erschwert, da diese Verbindungen in gesteigertem Maß luftempfindlich und thermisch sowie photochemisch instabil sind. Wünschenswert wären andere Quellen für Wasserstoffselenid und Wasserstofftellurid, die eine rückstandsfreie Gewinnung von Selenid-, Hydrogenselenid-, Di- und Polyselenidhaltigen ionischen Flüssigkeiten sowie Tellurid-, Hydrogentellurid-, Di- und Polytelluridhaltigen ionischen Flüssigkeiten garantieren.
Weiterhin ist nicht sichergestellt, dass der Anionenaustausch mit einem Alkalisulfid oder Alkali-Wasserstoffsulfid vollständig verläuft. Die weiteren Reinigungsoperationen verbrauchen dann viel Lösungsmittel, da ionische Flüssigkeiten aufgrund ihres geringen Dampfdrucks nicht besonders gut verdampft werden können und sich in der Regel bei thermischer Belastung im Aufreinigungsprozess zersetzen. Besonders bei stark hygroskopischen ionischen Flüssigkeiten ist es schwierig Restspuren an Wasser aus der Substanz zu entfernen.
Ein Anionenaustausch der auf einer Fällung von Erdalkali- oder Alkalimetallhalogeniden basiert z.B. als NaCl bewirkt zudem Verunreinigungen mit dem Fällungsmittel. Das ist besonders für elektrochemische Anwendungen ungünstig, da bei elektrochemischen Abscheidungen das Fällungsmittel unerwünschte Legierungen oder Bedeckungen der Oberfläche ergeben kann. Das kann relevante Eigenschaften der Beschichtung verändern.

## [Aufgabe]

Aufgabe der vorliegenden Erfindung ist es, die Nachteile des Standes der Technik zu beseitigen.

## [Lösung der Aufgabe]

Diese Aufgabe wird erfindungsgemäß gelöst, indem man eine ionische Flüssigkeit mit einem Organocarbonat-Anion und eine Chalkogenid-Verbindung zur Reaktion bringt und nach Decarboxylierung des Organocarbonat-Anions die ionische Flüssigkeit mit einem Chalkogenid-Anion vorliegt.

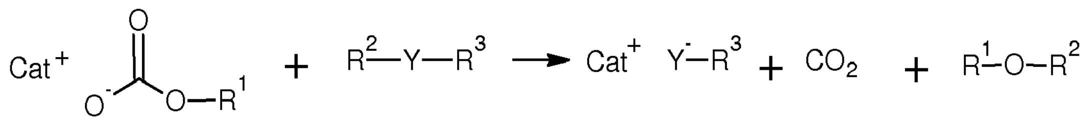

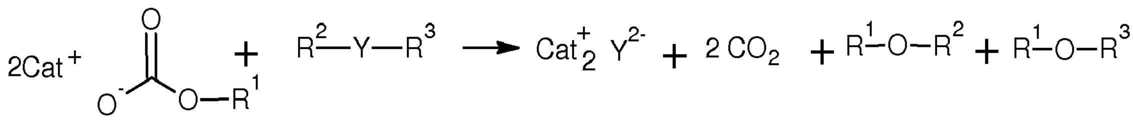

Dieses Verfahren hat den Vorteil, dass das Organocarbonat-Anion sich zersetzt und flüchtiges Kohlendioxid abspaltet. Das Organocarbonat-Anion stellt damit eine flüchtige Abgangsgruppe dar, so dass das Reaktionsgleichgewicht vollständig auf der Seite des Chalkogenid-Anions liegt. Die Aufreinigung ist damit wesentlich einfacher, da das Kohlendioxid aus der Lösung entweicht. Der sich bildende, vorzugsweise leichtflüchtige Alkohol $R^1$-OH oder Organosilylether $R^1$-$OSiR^aR^bR^c$ kann rückstandsfrei abgedampft werden, oder er verbleibt als unreaktive Komponente in der Lösung der chalkogenidhaltigen ionischen Flüssigkeit, falls diese in organischen Lösungsmitteln für weitere Umsetzungen eingesetzt wird.

Die organische Gruppe $R^1$ des Organocarbonat-Anion umfasst eine lineare oder verzweigte acyclische oder cyclische Alkylgruppe oder eine Arylgruppe mit einer Kohlenstoffanzahl von 1 bis 12, welche an das Carbonat gebunden ist. Die lineare oder verzweigte acyclische oder cyclische Alkylgruppe mit einer Kohlenstoffanzahl von 1 bis 12, umfasst beispielsweise, aber nicht einschränkend die Gruppen: Methyl, Ethyl, n-Propyl, iso-Propyl, n-Butyl, iso-Butyl, sec-Butyl, tert-Butyl, n-Pentyl, iso-Pentyl, sec-Pentyl, neo-Pentyl, 1,2-Dimethylpropyl, iso-Amyl, n-Hexyl, iso-Hexyl, sec-Hexyl, n-Heptyl, iso-Heptyl, n-Octyl, n-Decyl, 1-Nonyl, 1-Decyl, 1-Undecyl, 1- Dodecyl sowie allen Isomeren von Hexyl, Heptyl, Octyl, Nonyl, Decyl, Undecyl, Dodecyl.
Als Arylgruppe dienen Phenyl, Naphtyl oder subsubstituierte Derivate derselben.
R1 umfasst zusätzlich Wasserstoff.
Bevorzugt wird die ionische Flüssigkeit als Methylcarbonat eingesetzt.

Die Chalkogenid-Verbindung umfasst ein Chalkogen Y und die Gruppen $R^2$ und $R^3$.

Das Chalkogen Y oder die Chalkogen-Komponente Y umfasst Schwefel, Selen, Tellur und Kombinationen dieser.

Die Gruppen $R^2$ und $R^3$ umfassen unabhängig voneinander, aber nicht einschränkend Wasserstoff, Polychalkogenid, Organosilyl $R^aR^bR^cSi$- mit drei gleichen oder unterschiedlichen organischen Radikalen, alternativ auch Wasserstofforganosilyl $R^aR^bHSi$- und Kombinationen dieser. Besonders bevorzugte Kombinationen sind $H_2Y$, $(Me_3Si)_2Y$ und $Me_3Si$-Y-H.

Zur Herstellung des Wasserstoffchalkogenids mit organischem Kation $Cat^+$ erfolgt bevorzugt eine Protonierung des Organocarbonat-Anions O-CO-$OR^1$ mit anschließender Decarboxylierung und Bildung des Alkohols $R^1$-OH. Zur Herstellung des Organosilyl-chalkogenids erfolgt bevorzugt eine Desilylierung des Bis(organosilyl)chalkogenids durch das Organocarbonat-Anion O-CO-$OR^1$ gefolgt von einer Decarboxylierung und Bildung eines Silylethers $R^1$-O-$SiR^aR^bR^c$.

Wasserstoffchalkogenide als Chalkogenid-Verbindung sind auch durch Protolyse von Alkalichalkogenid oder Erdalkalichalkogenid oder Bis(organosilyl)chalkogeniden in Wasser, Alkoholen, oder anderen Protonensäuren erhältlich. Als Protonensäure gegenüber Organocarbonat kann auch ein N-H-funktionelles Ammoniumion, etwa $NH_4^+$ oder $[R^aR^bR^cRNH]^+$ (bzgl. $R^a$,$R^b$,$R^c$ - siehe weiter unten) dienen, eingesetzt beispielsweise als Organohydrogenammonium-Hydrogenchalkogenid. Bevorzugt erfolgt die Herstellung von Selenwasserstoff oder Tellurwasserstoff über das entsprechende Bis(trimethylsilyl)selenid oder das Bis(trimethylsilyl)tellurid in Methanol.

Zur Herstellung von Organosilylchalkogenid-Anionen wird das entsprechende Bis(organosilyl)chalkogenid bevorzugt in polaren aprotischen Lösungsmitteln z.B. Acetonitril umgesetzt. Das nucleophile Organocarbonat-Anion desilyliert das Bis(organosilyl)chalkogenid zum Organosilylchalkogenid-Anion. Es entsteht intermediär das Organosilylorganyl-carbonat, das unter Decarboxylierung zu Kohlenstoffdioxid und dem Organolsilyl-organylether zerfällt. Bei vollständig aprotischen Bedingungen entsteht im Reaktionsverlauf kein Alkohol.

Die organische Gruppe im Bis(organosilyl)chalkogenid, im Organosilylchalkogenid und Organohydrogenammonium-Hydrogenchalkogenid umfasst Wasserstoff oder eine lineare oder verzweigte acyclische oder cyclische Alkylgruppe mit einer Kohlenstoffanzahl von 1 bis 20. Die lineare oder verzweigte acyclische oder cyclische Alkylgruppe mit einer Kohlenstoffanzahl von 1 bis 20 umfasst beispielsweise, aber nicht einschränkend die Gruppen: Methyl, Ethyl, n-Propyl, iso-Propyl, n-Butyl, iso-Butyl, sec-Butyl, tert-Butyl, n-Pentyl, iso-Pentyl, sec-Pentyl, neo-Pentyl, 1,2-Dimethylpropyl, iso-Amyl, n-Hexyl, iso-Hexyl, sec-Hexyl, n-Heptyl, iso-Heptyl, n-Octyl, n-Decyl, 1-Nonyl, 1-Decyl, sowie allen Isomeren von Hexyl, Heptyl, Octyl, Nonyl, Decyl, Undecyl, Dodecyl, Tridecyl, Tetradecyl, Pentadecyl, Hexadecyl, Heptadecyl, Octadecyl, Nonadecyl und Eicosyl.

Weiterhin umfasst die organische Gruppe im Bis(organosilyl)chalkogenid oder im Organosilylchalkogenid bis zu drei unabhängig voneinander lineare oder verzweigte acyclische oder cyclische Alkylgruppen mit einer Kohlenstoffanzahl von 1 bis 20 (oben beschrieben) und oder Wasserstoff. Daher sind auch Kombinationen von unterschiedlichen oben genannten Alkylgruppen oder Wasserstoff am Silizium einsetzbar, z.B. Bis((Methyl, Ethyl, Propyl)Silyl) chalkogenid $(R^aR^bHSi)_2Y$. Bevorzugt wird Bis(trimethylsilyl)chalkogenid z.B. Bis(trimethylsilyl)sulfid, Bis(trimethylsilyl)selenid oder Bis(trimethylsilyl)tellurid als Chalkogenid-Verbindung eingesetzt.

Weiterhin umfasst die organische Gruppe im Organohydrogenammonium-Hydrogenchalkogenid bis zu drei unabhängig voneinander lineare oder verzweigte acyclische oder cyclische Alkylgruppen mit einer Kohlenstoffanzahl von 1 bis 20 (oben beschrieben) und oder Wasserstoff. Daher sind auch Kombinationen von unterschiedlichen oben genannten Alkylgruppen oder Wasserstoff am Silizium einsetzbar, z.B. $NH_4$ SH oder $NH_2(CH_3)_2$ SH.

Das Chalkogenid-Anion $Y^-$-$R^3$ umfasst beispielsweise, aber nicht einschränkend Sulfid, Selenid, Tellurid, Hydrogensulfid, Hydrogenselenid, Hydrogentellurid, Organosilylsulfid, Organosilylselenid, Organosilyltellurid, Polysulfid, Polyselenid, Polytellurid und Kombinationen dieser.

Die Herstellung der Chalkogenid-Anionen erfolgt bevorzugt mit einem Lösungsmittel. Eine Darstellung ohne Lösungsmittel ist ebenfalls möglich, da das entstehende Kohlendioxid abgedampft wird. Der entstehende Alkohol wird abgedampft oder durch die Chalkogenid-Verbindung abgefangen.

Die ionische Flüssigkeit besteht weiterhin aus einem Kation $Cat^+$. Das Kation weist Strukturen mit ionischem Stickstoff, Kohlenstoff, Phosphor und / oder Schwefel auf.
Diese Strukturen des Kations umfassen substituierte Strukturen wie beispielsweise, aber nicht einschränkend Imidazol, Pyridin, Guanidin, Pyrrolidin, Piperidin, Pyrazol Verbindungen mit Ammonium-Ion, Phosphonium-Ion, Sulfonium-Ion oder Sulfoxoium-Ion.

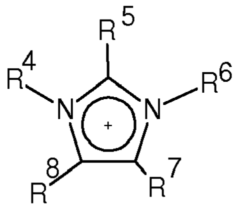
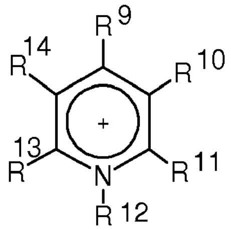
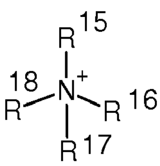
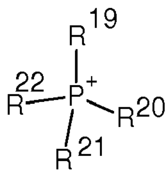
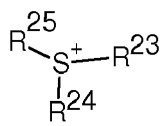

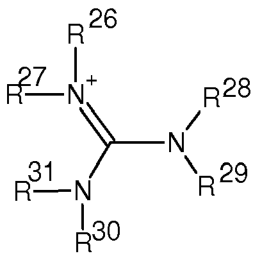
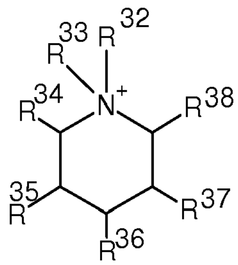
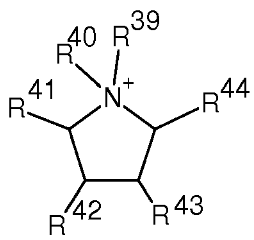
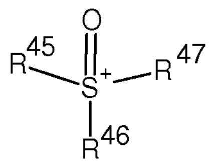

Die Gruppen $R^4$, $R^5$, $R^6$, $R^7$, $R^8$, $R^9$, $R^{10}$, $R^{11}$, $R^{12}$, $R^{13}$, $R^{14}$, $R^{15}$, $R^{16}$, $R^{17}$, $R^{18}$, $R^{19}$, $R^{20}$, $R^{21}$, $R^{22}$, $R^{23}$, $R^{24}$, $R^{25}$, $R^{26}$, $R^{27}$, $R^{28}$, $R^{29}$, $R^{30}$, $R^{31}$, $R^{32}$, $R^{33}$, $R^{34}$, $R^{35}$, $R^{36}$, $R^{37}$, $R^{38}$, $R^{39}$, $R^{40}$, $R^{41}$, $R^{42}$, $R^{43}$, $R^{44}$, $R^{45}$, $R^{46}$, $R^{47}$ umfassen unabhängig voneinander Wasserstoff oder eine lineare oder verzweigte acyclische oder cyclische Alkylgruppe mit einer Kohlenstoffanzahl von 1 bis 20, welche als Substituent an die substituierte Struktur gebunden ist. Die lineare oder verzweigte acyclische oder cyclische Alkylgruppe umfasst beispielsweise, aber nicht einschränkend die Gruppen: Methyl, Ethyl, n-Propyl, iso-Propyl, n-Butyl, iso-Butyl, sec-Butyl, tert-Butyl, n-Pentyl, iso-Pentyl, sec-Pentyl, neo-Pentyl, 1,2-Dimethylpropyl, iso-Amyl, n-Hexyl, iso-Hexyl, sec-Hexyl, n-Heptyl, iso-Heptyl, n-Octyl, n-Decyl, 1-Nonyl, 1-Decyl, sowie allen Isomeren von Hexyl, Heptyl, Octyl, Nonyl, Decyl, Undecyl, Dodecyl, Tridecyl, Tetradecyl, Pentadecyl, Hexadecyl, Heptadecyl, Octadecyl, Nonadecyl und Eicosyl.

Weiterhin umfassen $R^4$ $R^5$, $R^6$, $R^7$, $R^8$, $R^9$, $R^{10}$, $R^{11}$, $R^{12}$, $R^{13}$, $R^{14}$, $R^{15}$, $R^{16}$, $R^{17}$, $R^{18}$, $R^{19}$, $R^{20}$, $R^{21}$, $R^{22}$, $R^{23}$, $R^{24}$, $R^{25}$, $R^{26}$, $R^{27}$, $R^{28}$, $R^{29}$, $R^{30}$, $R^{31}$, $R^{32}$, $R^{33}$, $R^{34}$, $R^{35}$, $R^{36}$, $R^{37}$, $R^{38}$, $R^{39}$, $R^{40}$, $R^{41}$, $R^{42}$, $R^{43}$, $R^{44}$, $R^{45}$, $R^{46}$, $R^{47}$ unabhängig voneinander Gruppen wie eine Allyl- oder Vinylgruppe, eine Benzyl-, Aryl- oder Heteroarylgruppe.

Handelt es sich bei $R^4$, $R^5$, $R^6$, $R^7$, $R^8$, $R^9$, $R^{10}$, $R^{11}$, $R^{12}$, $R^{13}$, $R^{14}$, $R^{15}$, $R^{16}$, $R^{17}$, $R^{18}$, $R^{19}$, $R^{20}$, $R^{21}$, $R^{22}$, $R^{23}$, $R^{24}$, $R^{25}$, $R^{26}$, $R^{27}$, $R^{28}$, $R^{29}$, $R^{30}$, $R^{31}$, $R^{32}$, $R^{33}$, $R^{34}$, $R^{35}$, $R^{36}$, $R^{37}$, $R^{38}$, $R^{39}$, $R^{40}$, $R^{41}$, $R^{42}$, $R^{43}$, $R^{44}$, $R^{45}$, $R^{46}$, $R^{47}$ um eine Arylgruppe, so ist diese ausgewählt aus Phenyl, Naphthyl und Anthracenyl.

Handelt es sich bei $R^4$, $R^5$, $R^6$, $R^7$, $R^8$, $R^9$, $R^{10}$, $R^{11}$, $R^{12}$, $R^{13}$, $R^{14}$, $R^{15}$, $R^{16}$, $R^{17}$, $R^{18}$, $R^{19}$, $R^{20}$, $R^{21}$, $R^{22}$, $R^{23}$, $R^{24}$, $R^{25}$, $R^{26}$, $R^{27}$, $R^{28}$, $R^{29}$, $R^{30}$, $R^{31}$, $R^{32}$, $R^{33}$, $R^{34}$, $R^{35}$, $R^{36}$, $R^{37}$, $R^{38}$, $R^{39}$, $R^{40}$, $R^{41}$, $R^{42}$, $R^{43}$, $R^{44}$, $R^{45}$, $R^{46}$, $R^{47}$ um eine Heteroarylgruppe, so ist diese ausgewählt aus Furanyl, Pyrrolyl, Thiophenyl, Imidazolyl, Pyrazolyl, Thiazolyl, Oxazolyl, Isooxazolyl, einem Oxadiazolyl, Pyridinyl, Pyrazinyl, Pyrimidinyl, Pyridazinyl, einem Triazinyl, einem Tetrazinyl, 1,4-Dioxinyl, einem Thiazinyl, einem Oxazinyl, einem Azepinyl, einem Diazepinyl, Benzofuranyl, Isobenzofuranyl, Indolyl, Isoindolyl, Benzothiophenyl, Benzo[c]thiophenyl, Benzimidazolyl, Purinyl, Indazolyl, Benzoxazolyl, Benzisoxazolyl, Benzothiazolyl, Chinolinyl, Isochinolinyl, Chinoxalinyl, Acridinyl, Chinazolinyl oder Cinnolinyl.

Handelt es sich bei $R^4$, $R^5$, $R^6$, $R^7$, $R^8$, $R^9$, $R^{10}$, $R^{11}$, $R^{12}$, $R^{13}$, $R^{14}$, $R^{15}$, $R^{16}$, $R^{17}$, $R^{18}$, $R^{19}$, $R^{20}$, $R^{21}$, $R^{22}$, $R^{23}$,

$R^{24}$, $R^{25}$, $R^{26}$, $R^{27}$, $R^{28}$, $R^{29}$, $R^{30}$, $R^{31}$, $R^{32}$, $R^{33}$, $R^{34}$, $R^{35}$, $R^{36}$, $R^{37}$, $R^{38}$, $R^{39}$, $R^{40}$, $R^{41}$, $R^{42}$, $R^{43}$, $R^{44}$, $R^{45}R^{46}$, $R^{47}$ um eine Allyl- oder Benzylgruppe, so kann diese optional durch eine oder mehrere lineare oder verzweigte acyclische oder cyclische Alkylgruppen mit je 1 bis 20 Kohlenstoffatomen oder eine Alkenylgruppe, insbesondere eine Vinyl- oder Allylgruppe, substituiert sein.

Die linearen oder verzweigten acyclischen oder cyclischen Alkylgruppen mit 1 bis 20 Kohlenstoffatomen sowie die Aryl-, Heteroaryl-, Allyl- und Benzylgruppen können auch noch weitere funktionalisierte Kohlenwasserstoffreste tragen. Beispielhaft, aber nicht erschöpfend, seien folgende funktionelle Gruppen genannt:

- -CN, -F, -Cl, -Br, -I, $-SO_3^-$,
- primäre, sekundäre und tertiäre Aminogruppen $-NH_2$, $-NHR^a$, $-NR^aR^b$,
- $-OR^a$, -OH, $-COR^a$, -CHO,
- $-Si(OR^a)(OR^b)(OR^c)$, $-Si(OR^a)(OR^b)R^c$, $-SiR^aR^bR^c$,

$R^a$, $R^b$ und $R^c$ stehen unabhängig voneinander für einen organischen Rest mit vorzugsweise 1 bis 20 Kohlenstoffatomen, besonders bevorzugt für einen Aryl-, Heteroaryl-, Allyl-, Benzyl-, Vinyl- oder Alkylrest und ganz besonders bevorzugt für einen aliphatischen Rest, insbesondere eine lineare oder verzweigte acyclische oder cyclische Alkylgruppe mit einer Kohlenstoffanzahl von 1 bis 12, wie sie weiter oben definiert wurde. Optional können die Reste $R^a$, $R^b$, $R^c$ ganz oder teilweise fluoriert sein.

Enthalten die funktionellen Gruppen ihrerseits Kohlenstoffatome, beispielsweise im Falle von Ether-, Ester- oder Carbonylgruppen, so werden diese Kohlenstoffatome bei der Berechnung der Gesamtzahl von 1 bis 20 Kohlenstoffatomen der Alkylgruppen nicht berücksichtigt.

Alternativ können innerhalb der substituierten Strukturen d.h. zwischen den Gruppen $R^4$, $R^5$, $R^6$, $R^7$, $R^8$, zwischen $R^9$, $R^{10}$, $R^{11}$, $R^{12}$, $R^{13}$, $R^{14}$ zwischen $R^{15}$, $R^{16}$, $R^{17}$, $R^{18}$ zwischen $R^{19}$, $R^{20}$, $R^{21}$, $R^{22}$ zwischen $R^{23}$, $R^{24}$, $R^{25}$ zwischen $R^{26}$, $R^{27}$, $R^{28}$, $R^{29}$, $R^{30}$, $R^{31}$ zwischen $R^{32}$, $R^{33}$, $R^{34}$, $R^{35}$, $R^{36}$, $R^{37}$, $R^{38}$ zwischen $R^{39}$, $R^{40}$, $R^{41}$, $R^{42}$, $R^{43}$, $R^{44}$, zwischen $R^{45}$, $R^{46}$, $R^{47}$ zwei Gruppen unabhängig von den anderen Gruppen durch eine lineare oder verzweigte Alkylengruppe mit 2 bis 8 Kohlenstoffatomen verbunden sein. Somit stellen die zwei verbundenen Gruppen eine cyclische Gruppe, ausgewählt aus beispielsweise, aber nicht einschränkend Ethyl, n-Propyl, iso-Propyl, n-Butyl, iso-Butyl, sec-Butyl, n-Pentyl, iso-Pentyl, sec-Pentyl, 1,2-Dimethylpropyl, iso-Amyl, n-Hexyl, iso-Hexyl, sec-Hexyl, n-Heptyl, iso-Heptyl, sec-Heptyl, n-Octyl, iso-Octyl, sec-Octyl, dar. Die substituierte Struktur kann bei einer entsprechenden Vielzahl von Substituenten mehrere voneinander unabhängige cyclische Gruppen aufweisen.
In allen genannten Alkylengruppen kann eine $-CH_2$-Einheit optional durch O, NH, $NR^a$ ersetzt sein, worin $R^a$ wie oben definiert ist.

Die Alkylengruppen können, wie oben ausgeführt, linear oder verzweigt sein. Vorteilhafte lineare Alkylengruppen sind beispielsweise die 1,2-Ethylen- und die 1,3-Propylengruppe. Eine vorteilhafte verzweigte Alkylengruppe ist die 1,2-Propylengruppe.
Die Alkylengruppen cyclischer Derivate können des Weiteren die oben bereits aufgeführten weiteren funktionelle Gruppen funktionalisierter Kohlenwasserstoffreste tragen. Besonders vorteilhaft tragen die Alkylengruppen NH-, Ester, CN- oder Carbonylgruppen.

Weiterhin sind die für $R^4$, $R^5$, $R^6$, $R^7$, $R^8$, $R^9$, $R^{10}$, $R^{11}$, $R^{12}$, $R^{13}$, $R^{14}$, $R^{15}$, $R^{16}$, $R^{17}$, $R^{18}$, $R^{19}$, $R^{20}$, $R^{21}$, $R^{22}$, $R^{23}$, $R^{24}$, $R^{25}$, $R^{26}$, $R^{27}$, $R^{28}$, $R^{29}$, $R^{30}$, $R^{31}$, $R^{32}$, $R^{33}$, $R^{34}$, $R^{35}$, $R^{36}$, $R^{37}$, $R^{38}$, $R^{39}$, $R^{40}$, $R^{41}$, $R^{42}$, $R^{43}$, $R^{44}$, $R^{45}$, $R^{46}$, $R^{47}$ aufgezählten Gruppen ein-zeln und an unterschiedlichen Substitutionsstellen in Kombination einsetzbar.

Zur Herstellung des Chalkogenid-Anions aus einer organocarbonathaltigen ionischen Flüssigkeit, wird die organocarbonathaltige ionische Flüssigkeit bevorzugt durch Umsetzung einer substituierten Struktur (siehe oben), der ein Substituent fehlt, mit einem Methylorganocarbonat hergestellt.
Die organische Gruppe des Organocarbonats umfasst eine lineare oder verzweigte acyclische oder cyclische Alkylgruppe mit einer Kohlenstoffanzahl von 1 bis 12, welches an das Methylcarbonat gebunden ist. Die lineare oder verzweigte acyclische oder cyclische Alkylgruppe mit einer Kohlenstoffanzahl von 1 bis 12, umfasst beispielsweise, aber nicht einschränkend die Gruppen: Methyl, Ethyl, n-Propyl, iso-Propyl, n-Butyl, iso-Butyl, sec-Butyl, tert-Butyl, n-Pentyl, iso-Pentyl, sec-Pentyl, neo-Pentyl, 1,2-Dimethylpropyl, iso-Amyl, n-Hexyl, iso-Hexyl, sec-Hexyl, n-Heptyl, iso-Heptyl, n-Octyl, n-Decyl, 1-Nonyl, 1-Decyl, 1- Undecyl, 1-Dodecyl sowie allen Isomeren von Hexyl, Heptyl, Octyl, Nonyl, Decyl, Undecyl, Dodecyl.
Die organische Gruppe des Organocarbonats umfasst zusätzlich auch Wasserstoff, insbesondere da Hydrogencarbonat durch Hydrolyse von Organocarbonat entstehen kann.
Bevorzugt wird die die als Startmaterial dienende organocarbonathaltige ionische Flüssigkeit durch N-Methylierung von beliebig substituierten (vgl. Abb.) Imidazolen, Pyridinen, Guanidinen, Pyrrolidinen, Piperidinen, Pyrazolen, von nichtzyklischen primären, sekundären oder tertiären Aminen, durch P-Methylierung eines sekundären oder tertiären Phosphans oder S-Methylierung eines Diorganosulfans oder Diorganosulfoxids mit Dimethylcarbonat hergestellt. Derartige Methylierungen sind literaturbekannt. Das hier beanspruchte Verfahren umfasst aber auch Kombinationen des literaturbekannten Methylierungsschrittes mit den hier beanspruchten Folgereaktionen des Anionaustausches zu einem kombinierten Prozess.
Das kann als unmittelbar aufeinanderfolgende Reaktion in einem einzigen Reaktionsgefäß durchgeführt werden.
Als Edukt wird ein Kationenprecursor für ionische Flüssigkeiten vorgelegt. Dieser umfasst oben beschriebene substituierte Strukturen für Kationen, bei denen ein Substituent fehlt. Mit dem Methylorganocarbonat z.B. Dimethylcarbonat wird eine Methylierung des Kationenprecursors durchgeführt und anschließend in situ mit einer oben beschriebenen Chalkogenid-Verbindung umgesetzt. Es entsteht eine ionische Flüssigkeit mit einem Chalkogenid-Anion.
Es wurde überraschend festgestellt, dass ionische Flüssigkeiten mit einem Chalkogenid-Anion durch die Organocarbonat-Anion Zersetzung in einer hohen analytischen Reinheit herstellbar sind. Das Chalkogenid-Anion ist sehr nucleophil und ein guter Donorligand. Diese besonders hohe Reaktivität kann man auch dazu ausnutzen, um Metalle und ihre Metallverbindungen und Metallkomplexe $[MX_nL_{m]}$ (M ist ein Metall des s-, p-, d- oder f-Blocks des Periodensystems, X sind beliebige anionische Liganden, L beliebige Neutralliganden) bei Temperaturen unter 100°C z.B. Raumtemperatur in Metallchalkogende zu überführen. So ist es möglich binäre Metallsulfide, Metallselenide, Metalltelluride oder aber ternäre und multinäre Metallchalkogenide mit verschiedenen Metallen und verschiedenen Chalkogenidionen phasenrein und in amopher oder kristalliner Form, mit wenig Energieaufwand herzustellen.

Die beanspruchten ionischen Flüssigkeiten mit Chalkogenid-Anion sind unterhalb von ca. 180 °C, bevorzugt unterhalb von 100 °C flüsig. Einige der hier beanspruchten salzartigen Chalkogenide sind bei Raumtemperatur flüssig. Es wurde festgestellt, dass die Art und der Substitutionsgrat des organischen Kations $Cat^+$ einen entscheidenden Einfluss auf den Schmelzpunkt und die termische Zersetzungstemperatur der beanspruchten salzartigen Chalkogenid-Verbindungen haben.

Eine so hergestellte ionische Flüssigkeit ist daher unter anderem in folgenden Bereichen einsetzbar:

- als redoxaktiver Elektrolyt für Batterien, Akkumulatoren und andere elektrochemische Zellen,
- als Redoxmediator in Farbstoffsolarzellen und Quantum Dot sensibilisierten Solarzellen,
- zur Beschichtung von Wafern oder Substraten mit Metall-Chalkogenid-Materialien,
- als Lösungsmittel für chemische Reaktionen,
- als Lösungsmittel und reaktive Komponente für elementare Formen der Chalkogene unter Bildung von ionischen Flüssigkeiten mit Polychalkogenidanionen
- als reaktive Komponente in Fällungsreaktionen binärer oder multinärer Metallchalkogenide $M_nM'_mS_xSe_yTe_z$ in organischen Lösungsmitteln oder in der reaktiven ionischen Flüssigkeit im Sinne einer Schmelzflusslonothermalsynthese,
- in der Herstellung von Metallchalkogenid-basierten Dünnfilm-Halbleitermaterialien $M_nM'_mS_xSe_yTe_z$ aus Lösung,
- in der Herstellung von Metallchalkogeniden, Kombinationen dieser oder Dünnfilm-Halbleitermaterialien $M_nM'_mS_x$-$Se_yTe_z$ im Sinne einer Printed Electronics Strategie, wobei die verschiedenen Chalkogenkomponenten in Form verschiedener Lösungen der hier beanspruchten Chalkogenidhaltigen ionischen Flüssigkeiten auf dem Target mit Lösungen reaktiver Metallkomponenten über verschiedene Coating-, Spray- und Print-Verfahren zur Reaktion gebracht werden.

In $M_nM'_mS_xSe_yTe_z$ stellen dabei M und M' unabhängig voneinander Metalle des s-Blocks, p-Blocks, d-Blocks oder f-Blocks (z.B. Li, Na, K, Rb, Be, Mg, Ca, Sr, Ba, Sc, Y, La, Ac, Ti, Zr, Hf, V, Nb, Ta, Cr, Mo, W, Mn, Re, Fe, Ru, Os, Co, Rh, Ir, Ni, Pd, Pt, Cu, Ag, Au, Zn, Cd, Hg, Al, Ga, In, Tl, Si, Ge, Sn, Bi, Eu, Gd, La, Ce, Pr, Nd, Sm, TB, Dy, Ho, Er, Tm, Yb, Lu) und die Indizes n,m,x,y,z ganze oder gebrochene Zahlen dar.

Die erfindungsgemäße Ausführung ist nachfolgend erläutert, wobei die Erfindung alle nachfolgend aufgeführten bevorzugten Ausführungsformen einzeln und in Kombination umfasst.

**[Ausführungsbeispiele]**

Die Herstellung der ionischen Flüssigkeiten kann nach mehreren Verfahren laufen.

**Allg. Herstellung der erfindungsgemäßen Substanzen**

Die ionische Flüssigkeit wird als Methylcarbonat in einer 5 bis 80%igen bevorzugt 10 bis 50%igen Lösung mit einem Lösungsmittel eingesetzt und auf Arbeitstemperatur im Bereich von -50 bis 80 °C bevorzugt -10 bis 30 °C gebracht. Die Chalkogenid-Verbindung wird bevorzugt stöchiometrisch oder im Überschuss von bis zu 20% mehr als die ionische Flüssigkeit zudosiert. Nach 1 bis 60 Minuten bevorzugt 20 bis 30 Minuten wird aufgearbeitet.
Die Aufarbeitung ist in mehreren Varianten durchführbar. Beispielsweise aber nicht darauf beschränkt wurden folgende Varianten gewählt:

Variante 1: Einengen der Lösung
Variante 2: Einengen der Lösung mit anschließender Umkristallisation aus Acetonitril und Diethylether.
Variante 3: Einengen der Lösung mit anschließender Umkristallisation aus Acetonitril und Diethylether bei -80 bis 0 °C, bevorzugt -30°C.

Für alle im Folgenden beschriebenen erfindungsgemäßen Substanzen konnten passende Elementaranalysen (C, H, (N), (S)) erhalten werden.

**Darstellung von 1-Ethyl-3-methyl-imidazolium-hydrogensulfid ([EMIM]SH)**

In 26.9 g eine 30%igen Lösung von EMIM-methylcarbonat in Methanol (43.4 mmol) wurde Schwefelwasserstoff eingeleitet. Die Lösung wurde im Feinvakuum eingeengt, der Rückstand im Vakuum getrocknet und aus einem Acetonitril-Diethylether-Gemisch bei -30 °C umkristallisiert.
Ausbeute: 4.62 g (32.0 mmol, 74%).
$^1$H-NMR (300.1 MHz, $CD_3CN$): δ=-3.54 (s, 1H, S*H*), 1.41 (t, $^3J_{HH}$ = 7.3 Hz, 3H, $CH_2CH_3$), 3.90 (s, 3H, N*Me*), 4.26 (q, $^3J_{HH}$=7.3 Hz , 2H, $CH_2CH_3$), 7.69 (d, $^3J_{HH}$ = 1.9 Hz, 1 H, *H4/5*), 7.80 (d, $3J_{HH}$ = 1.9 Hz, 1 H, *H4/5*), 10.66 (s, 1 H, *H2*) ppm. $^{13}$C-NMR (75.5 MHz, $CD_3CN$): δ = 15.6 (1 C, $CH_2CH_3$), 36.4 (1 C, $CH_2CH_3$), 45.2 (1 C, N*Me*), 122.6 (1 C, *C4/5*), 124.0 (1 C, *C4/5*), 138.0 (1 C, *C2*) ppm.

Kristallstruktur siehe Fig. 1

Kristalldaten:

Kristall: farblose Nadel (0.3 x 0.1 x 0.1 mm3)
Kristallisiert in der monoklinen Raumgruppe P21/n mit 4 Formeleinheiten pro Elementarzelle.
Zelle:

$$a = 8.6101(12)\ \text{Å},\ b = 7.6972(8)\ \text{Å},\ c = 12.779(2)\ \text{Å},$$

$$\alpha = \gamma = 90°, \beta = 107.878(13)°,$$

$$V = 806.0(2)\ \text{Å}^3.$$

**Darstellung von N-Butyl-N-methyl-pyrrolidinium-hydrogensulfid ([BMPyr]SH)**

*N*-Butyl-*N*-methyl-pyrrolidinium-methylcarbonat (15.1 g, 69.5 mmol) wurde in Methanol (40 mL) gelöst und Schwefelwasserstoff in die Lösung eingeleitet. Die Lösung wurde im Feinvakuum eingeengt, der Rückstand im Vakuum getrocknet und aus einem Acetonitril-Diethylether-Gemisch bei -30 °C umkristallisiert.
Ausbeute: 5.65 g (32.2 mmol, 46%).
$^1$H-NMR (300.1 MHz, $CD_3CN$): δ =-3.83 (s, 1H, S*H*), 0.93 (t, $^3J_{HH}$ = 7.3 Hz, 3H, $CH_2CH_3$), 1.28-1.40 (m, 2H, $CH_2CH_3$), 1.65-1.75 (m, 2H, $NCH_2CH_2$), 2.09-2.17 (m, 4H, $CH_2$*(3,4)*), 3.07 (s, 3H, N*Me*), 3.44-3.49 (m, 2H, $NCH_2CH_2$), 3.56-3.62 (m, 4H, $CH_2$*(2,5)*) ppm.
$^{13}$C-NMR (75.5 MHz, $CD_3CN$): δ = 13.8 (1 C, $CH_2CH_3$), 20.3 (1 C), 22.1 (1 C), 26.2 (1 C), 48.8 (t, $^3J_{CN}$ = 3.9 Hz, 2C, *C3/4),* 64.3 (t, $^2J_{CN}$ = 2.8 Hz, 2C, *C2/5*), 64.8 (t, $^2J_{CN}$ = 3.1 Hz, 1C)) ppm.

Kristallstruktur siehe Fig. 2

Kristalldaten:

Kristall: farblose Nadel (0.38 x 0.14 x 0.12 $mm^3$)
Kristallisiert in der tetragonalen Raumgruppe *P*42*bc* mit 8 Formeleinheiten pro Elementarzelle.
Zelle:

$$a = b = 15.6635(6)\ \text{Å},\ c = 8.9408(5)\ \text{Å},$$

$$\alpha = \beta = \gamma = 90°$$

$$V = 2193.6(2)\ \text{Å}^3.$$

**Darstellung von N-Butyl-N-methyl-piperidinium-hydrogensulfid ([BMPip]SH)**

*N*-Butyl-*N*-methyl-piperidinium-methylcarbonat (1.43 g, 6.18 mmol) wurde in Methanol (7 mL) gelöst und Schwefelwasserstoff in die Lösung eingeleitet. Die Lösung wurde im Feinvakuum eingeengt, der Rückstand im Vakuum getrocknet und aus einem Acetonitril-Diethylether-Gemisch umkristallisiert.
Ausbeute: 0.91 g (4.8 mmol, 78%).
$^1$H-NMR (300.1 MHz, $CD_3CN$): $\delta$= -3.78 (s, 1H, S*H*), 0.96 (t, $^3J_{HH}$ = 7.4 Hz, 3H, $CH_2CH_3$), 1.34-1.41 (m, 2H, $CH_2CH_3$), 1.58-1.71 (m, 4H), 1.80-1.86 (m, 4H), 3.00 (s, 3H, NMe), 3.31-3.39 (m, 6H) ppm.
$^{13}$C-NMR (75.5 MHz, $CD_3CN$): $\delta$ = 13.8 (1 C), 20.4 (1 C), 20.5 (1 C), 21.6 (1 C), 24.3 (1 C), 48.5 (br s, 2C), 61.7 (1 C), 63.9 (br s, 2C) ppm.

**Darstellung von Methyl-tri-isopropyl-phosphonium-hydrogensulfid ([MeP(iPr)$_3$]SH)**

Methyl-tri-*iso*propyl-phosphonium-methylcarbonat (3.11 g, 12.4 mmol) wurde in Methanol (20 mL) gelöst und Schwefelwasserstoff in die Lösung eingeleitet. Die Lösung wurde im Feinvakuum eingeengt, der Rückstand im Vakuum getrocknet und aus einem Acetonitril-Diethylether-Gemisch bei -8 °C umkristallisiert.
Ausbeute: 1.52 g (7.31 mmol, 59%).
$^1$H-NMR (300.1 MHz, $CD_3CN$): $\delta$ = -3.86 (s, 1H, S*H*), 1.30 (dd, $^3J_{HH}$ = 7.3 Hz, $^3J_{HP}$ = 16.2 Hz, 18H, $CH(CH_3)_2$), 1.81 (d, $^2J_{HP}$ = 12.6 Hz, 3H, $PCH_3$), 2.79 (dsept, $^3J_{HH}$ = 7.2 Hz, $^2J_{HP}$ = 12.2 Hz, 3H, $CH(CH_3)_2$) ppm.
$^{13}$C-NMR (75.5 MHz, $CD_3CN$): $\delta$ = -0.3 (d, $^1J_{CP}$ = 49.5 Hz, 1C, $PCH_3$), 16.8 (d, $^2J_{CP}$= 2.7 Hz, 6C, $CH(CH_3)_2$), 44.4 (d, $^1J_{CP}$= 44.4 Hz, 3C, $PCH(CH_3)_2$) ppm.
$^{31}$P-NMR (101.3 MHz, $CD_3CN$): $\delta$ = 46.5 ppm.

**Darstellung von Methyl-tri-nbutyl-phosphonium-hydrogensulfid ([MeP(nBu)$_3$]SH)**

Methyl-tri-*n*butyl-phosphonium-methylcarbonat (23.4 g 80.0 mmol) wurde in Methanol (40 mL) gelöst und Schwefelwasserstoff in die Lösung eingeleitet. Die Lösung wurde im Feinvakuum eingeengt und der Rückstand anschließend in Acetonitril (30 mL) und Diethylether (50 mL) aufgenommen. Bei -80 °C wurden Acetonitril und das Produkt cokristallisiert, die überstehende Lösung abgehoben und der Rückstand im Feinvakuum getrocknet.
Ausbeute: 18.3 g (73.2 mmol, 91 %).
$^1$H-NMR (300.1 MHz, DMSO-$d_6$): $\delta$ = -4.04 (s, 1 H, S*H*), 0.90 (t, $^3J_{HH}$= 7.1 Hz, 9H, $CH_2CH_3$), 1.33-1.53 (m, 12H, $CH_2CH_2$), 1.85 (d, $^2J_{HP}$ = 14.1 Hz, 3H, $PCH_3$), 2.19-2.29 (m, 6H, $PCH_2$) ppm.
$^{13}$C-NMR (75.5 MHz, DMSO-$d_6$): $\delta$ = 3.3 (d, $^1J_{CP}$ = 51.3 Hz, 1C, $PCH_3$), 13.2 (3C, $CH_2CH_3$), 18.9 (d, $^1J_{CP}$ = 49.2 Hz, 3C, $PCH_2$), 22.6 (d, $J_{CP}$ = 4.4 Hz, 3C, $CH_2$), 23.2 (d, $J_{CP}$ = 15.8 Hz, 3C, $CH_2$) ppm.
$^{31}$P-NMR (101.3 MHz, DMSO-$d_6$): $\delta$ = 34.0 ppm.

**Darstellung von 1-Ethyl-3-methyl-imidazolium-hydrogenselenid ([EMIM]SeH)**

3.68 g einer 30%igen Lösung von EMIM-methylcarbonat in Methanol (5.93 mmol, 1.00 eq) wurden auf 0 °C gekühlt und $TMS_2Se$ (1.63 g, 7.23 mmol, 1.22 eq) langsam zugetropft. Die Mischung wurde 30 Minuten bei 0 °C und 30 Minuten bei Raumtemperatur gerührt, bevor das Lösungsmittel im Feinvakuum entfernt wurde. Umkristallisation aus einem Acetonitril-Diethylether-Gemisch bei -30 °C lieferte die analysenreine Verbindung.
Ausbeute: 796 mg (4.16 mmol, 70%).
$^1H$-NMR (300.1 MHz, $CD_3CN$): $\delta$ = -6.38 (s, 1 H, Se*H),* 1.45 (t, $^3J_{HH}$ = 7.4 Hz, 3H, $CH_2CH_3$), 3.89 (s, 3H, NMe), 4.25 (q, $^3J_{HH}$ = 7.3 Hz , 2H, $CH_2CH_3$), 7.49 (m, 1 H, *H4/5),* 7.57 (m, 1 H, *H4/5),* 9.78 (s, 1 H, H2) ppm.
$^{13}C$-NMR (75.5 MHz, $CD_3CN$): $\delta$ = 15.6 (1 C, $CH_2CH_3$), 36.7 (1 C, $CH_2CH_3$), 45.5 (1 C, NMe), 122.7 (1 C, *C4/5),* 124.2 (1 C, *C4/5),* 137.4 (1 C, *C2)* ppm.

**Darstellung von N-Butyl-N-methyl-pyrrolidinium-hydrogenselenid ([BMPyr]SeH)**

*N*-Butyl-*N*-methyl-pyrrolidinium-methylcarbonat (1.06 g, 4.88 mmol, 1.00 eq) wurde in Methanol (5 mL) gelöst und die Lösung auf 0 °C gekühlt. $TMS_2Se$ (1.29 g, 5.72 mmol, 1.17 eq) wurde langsam zugetropft. Die Mischung wurde 30 Minuten bei 0 °C und 30 Minuten bei Raumtemperatur gerührt, bevor das Lösungsmittel im Feinvakuum entfernt wurde. Umkristallisation aus einem Acetonitril-Diethylether-Gemisch bei -30 °C lieferte die analysenreine Verbhdung.
Ausbeute: 635 mg (1.86 mmol, 59%).
$^1H$-NMR (300.1 MHz, $CD_3CN$): $\delta$ = -6.56 (s, 1H, Se*H*), 0.92 (t, $^3J_{HH}$ = 7.3 Hz, 3H, $CH_2CH_3$), 1.27-1.39 (m, 2H, $CH_2CH_3$), 1.64-1.75 (m, 2H, $NCH_2CH_2$), 2.09-2.17 (m, 4H, $CH_2$*(3,4)*), 3.06 (s, 3H, NMe), 3.44-3.49 (m, 2H, $NCH_2CH_2$), 3.55-3.62 (m, 4H, $CH_2$*(2,5)*) ppm.
$^{13}C$-NMR (75.5 MHz, DMSO-$d_6$): $\delta$ = 13.47 (1 C, $CH_2CH_3$), 19.2 (1 C, br), 21.0 (1 C), 24.9 (1C), 47.5 (t, $^3J_{CN}$ = 3.7 Hz, 2C, *C3/4),* 62.7 (br s, 2C, *C2/5),* 63.3 (t, $^2J_{CN}$ = 2.8 Hz, 1 C) ppm.

**Darstellung von N-Butyl-N-methyl-pyrrolidinium-hydrogentellurid ([BMPyr]TeH)**

*N*-Butyl-*N*-methyl-pyrrolidinium-methylcarbonat (635 mg, 2.92 mmol, 1.00 eq) wurde in Methanol (8 mL) gelöst und die Lösung auf 0 °C gekühlt. $TMS_2Te$ (920 mg, 3.36 mmol, 1.15 eq) wurde langsam zugetropft. Die Mischung wurde 30 Minuten bei 0 °C und 30 Minuten bei Raumtemperatur gerührt, bevor das Lösungsmittel im Feinvakuum entfernt wurde.
Ausbeute: 530 mg (1.96 mmol, 67%).
$^1H$-NMR (300.1 MHz, $CD_3CN$): $\delta$ = -13.34 (s, 1 H, Te*H*), 0.94 (t, $^3J_{HH}$ = 7.3 Hz, 3H, $CH_2CH_3$), 1.30-1.42 (m, 2H, $CH_2CH_3$), 1.67-1.78 (m, 2H, $NCH_2CH_2$), 2.10-2.22 (m, 4H, $CH_2$*(3,4)*), 3.02 (s, 3H, NMe), 3.35-3.40 (m, 2H, $NCH_2CH_2$), 3.49-3.57 (m, 4H, $CH_2$*(2,5)*) ppm.
$^{13}C$-NMR (75.5 MHz, $CD_3CN$): $\delta$ = 13.7 (1C, $CH_2CH_3$), 20.2 (1C, br), 22.1 (1C), 26.1 (1 C), 49.3 (t, $^3J_{CN}$ = 4.0 Hz, 2C, *C3/4),* 64.6 (t, $^2J_{CN}$ = 2.8 Hz, 2C, *C2/5),* 65.2 (t, $^2J_{CN}$ = 3.1 Hz, 1 C)) ppm.

**Darstellung von Methyl-tri-nbutyl-phosphonium-hydrogentellurid ([MeP(nBu)$_3$]TeH)**

Methyl-tri-*n*butyl-phosphonium-methylcarbonat (991 mg 3.39 mmol, 1.00 eq) wurde in Methanol (6 mL) gelöst, die Lösung auf 0 °C gekühlt. $TMS_2Te$ (1.03 g, 3.76 mmol, 1.11 eq) wurde tropfenweise zugegeben. Die Mischung wurde eine halbe Stunde bei 0 °C gerührt, dann über einen Sprtzenfilter filtriert und die Lösung weitere 30 Minuten bei 0 °C gerührt. Die Mischung wurde anschließend eine halbe Stunde bei Raumtemperatur gerührt und schließlich im Feinvakuum eingeengt.
Ausbeute: 1.05 g, (3.04 mmol, 89%).
$^1H$-NMR (300.1 MHz, DMSO-$d_6$): $\delta$ = -13.06 (s, 1 H, Te*H*), 0.90 (t, $^3J_{HH}$ = 6.9 Hz, 9H, $CH_2CH_3$), 1.32-1.55 (m, 12H, $CH_2CH_2$), 1.85 (d, $^2J_{HP}$ = 14.0 Hz, 3H, P*Me*), 2.16-2.30 (m, 6H, $PCH_2$) ppm.
$^{13}C$-NMR (75.5 MHz, DMSO-$d_6$): $\delta$ = 3.5 (d, $^1J_{CP}$ = 51.3 Hz, 1C, $PCH_3$), 13.2 (3C, $CH_2CH_3$), 19.0 (d, $^1J_{CP}$ = 49.1 Hz, 3C, $PCH_2$), 22.6 (d, $J_{CP}$ = 4.3 Hz, 3C, $CH_2$), 23.2 (d, $J_{CP}$ = 15.9 Hz, 3C, $CH_2$) ppm.
$^{31}P$-NMR (101.3 MHz, DMSO-$d_6$): $\delta$ = 35.6 ppm.

**Darstellung von *N*-Butyl-*N*-methyl-pyrrolidinium-disulfid [BMPyr]$_2$S$_2$**

*N*-Butyl-*N*-methyl-pyrrolidinium-hydrogensulfid (304 mg, 1.73 mmol, 2.0 eq) und Schwefel (28 mg, 0.87 mmol, 1.0 eq) wurden vereint und mit Acetonitril (3 mL) versetzt. Unter Bildung von $H_2S$ löste sich der Schwefel vollständig auf. Die Mischung wurde 14 Stunden bei Raumtemperatur gerührt, das Lösungsmittel anschließend im Feinvakuum entfernt.
Ausbeute: Quantitativ.

$^1$H-NMR (300.1 MHz, DMSO-$d_6$): δ = 0.93 (t, $^3J_{HH}$= 7.3 Hz, 3H, $CH_2CH_3$), 1.26-1.38 (m, 2H, $CH_2CH_3$), 1.63-1.74 (m, 2H, $NCH_2CH_2$), 2.04-2.13 (m, 4H, $CH_2$*(3,4)*), 3.01 (s, 3H, NMe), 3.44-3.49 (m, 2H, $NCH_2CH_2$), 3.43-3.57 (m, 4H, $CH_2$*(2,5)*) ppm. $^{13}$C-NMR (75.5 MHz, DMSO-$d_6$): δ = 13.5 (1C, $CH_2CH_3$), 19.3 (1C), 21.1 (1C), 24.9 (1 C), 47.6 (br s, 2C, *C3/4),* 62.8 (br s, 2C, *C2/5),* 63.4 (br s, 1 C) ppm.

**Darstellung von *N*-Butyl-*N*-methyl-pyrrolidinium-hexasulfid $[BMPyr]_2S_6$**

*N*-Butyl-*N*-methyl-pyrrolidinium-hydrogensulfid (313 mg, 1.79 mmol, 2.00 eq) und Schwefel (143 mg, 4.46 mmol, 5.00 eq) wurden vereint und mit Acetonitril (3 mL) versetzt. Unter Bildung von $H_2S$ löste sich der Schwefel vollständig auf. Die Mischung wurde 14 Stunden bei Raumtemperatur gerührt, das Lösungsmittel anschließend im Feinvakuum entfernt.
Ausbeute: Quantitativ.
$^1$H-NMR (300.1 MHz, DMSO-$d_6$): δ = 0.93 (t, $^3J_{HH}$= 7.0 Hz, 3H, $CH_2CH_3$), 1.25-1.40 (br m, 2H, $CH_2CH_3$), 1.63-1.78 (br s, 2H, $NCH_2CH_2$), 2.04-2.18 (br s, 4H, $CH_2$*(3,4)*), 3.02 (s, 3H, N*Me*), 3.30-3.40 (m, 2H, $NCH_2CH_2$), 3.43-3.59 (m, 4H, $CH_2$*(2,5)*) ppm.
$^{13}$C-NMR (75.5 MHz, DMSO-$d_6$): δ = 13.5 (1C, $CH_2CH_3$), 19.3 (1C), 21.3 (1C), 25.0 (1 C), 48.2 (br s, 2C, *C3/4),* 63.2 (br s, 2C, *C2/5),* 63.9 (br s, 1 C) ppm.

**Darstellung von Methyl-tri-*n*butyl-phosphonium-disulfid $[MeP(nBu)_3]_2S_2$**

Methyl-tri-*n*butyl-phosphonium-hydrogensulfid (106mg, 423 μmol, 2eq) und Schwefel (7 mg, 0.2 mmol, 1 eq) wurden vereint und mit Acetonitril (1 mL) versetzt. Unter Bildung von $H_2S$ löste sich der Schwefel vollständig auf. Die Mischung wurde eine halbe Stunde bei Raumtemperatur gerührt, das Lösungsmittel anschließend im Feinvakuum entfernt.
Ausbeute: Quantitativ.
$^1$H-NMR (300.1 MHz, DMSO-$d_6$): δ = 0.90 (t, $^3J_{HH}$= 7.1 Hz, 9H, $CH_2CH_3$), 1.32-1.53 (m, 12H, $CH_2CH_2$), 1.84 (d, $^2J_{HP}$ = 14.0 Hz, 3H, $PCH_3$), 2.17-2.31 (m, 6H, $PCH_2$) ppm.
$^{13}$C-NMR (75.5 MHz, DMSO-$d_6$): δ = 3.1 (d, $^1J_{CP}$ = 51.4 Hz, 1C, $PCH_3$), 13.3 (3C, $CH_2CH_3$), 18.8 (d, $^1J_{CP}$ = 49.2 Hz, 3C, $PCH_2$), 22.7 (d, $J_{CP}$ = 4.4 Hz, 3C, $CH_2$), 23.2 (d, $J_{CP}$ = 16.0 Hz, 3C, $CH_2$) ppm.
$^{31}$P-NMR (101.3 MHz, DMSO-$d_6$): δ = 35.6 ppm.

**Darstellung von Methyl-tri-*n*butyl-phosphonium-tetrasulfid $[MeP(nBu)_3]_2S_4$**

Methyl-tri-nbutyl-phosphonium-hydrogensulfid (502 mg, 2.00 mmol, 2.00 eq) und Schwefel (97 mg, 3.0 mmol, 3.0 eq) wurden vereint und Acetonitril (3 mL) versetzt. Unter Bildung von $H_2S$ löste sich der Schwefel vollständig auf. Die Mischung wurde eine halbe Stunde bei Raumtemperatur gerührt, das Lösungsmittel anschließend im Feinvakuum entfernt.
Ausbeute: Quantitativ.
$^1$H-NMR (300.1 MHz, DMSO-$d_6$): δ = 0.91 (t, $^3J_{HH}$= 6.7 Hz, 9H, $CH_2CH_3$), 1.33-1.57 (m, 12H, $CH_2CH_2$), 1.87 (d, $^2J_{HP}$ = 14.0 Hz, 3H, $PCH_3$), 2.17-2.31 (m, 6H, $PCH_2$) ppm.
$^{13}$C-NMR (75.5 MHz, DMSO-$d_6$): δ = 3.8 (d, $^1J_{CP}$ = 50.7 Hz, 1C, $PCH_3$), 13.3 (3C, $CH_2CH_3$), 19.2 (d, $^1J_{CP}$ = 49.1 Hz, 3C, $PCH_2$), 22.7 (d, $J_{CP}$ = 4.0 Hz, 3C, $CH_2$), 23.3 (d, $J_{CP}$ = 15.9 Hz, 3C, $CH_2$) ppm.
$^{31}$P-NMR (101.3 MHz, DMSO-$d_6$): δ = 35.7 ppm.

**Darstellung von 1-Ethyl-3-methyl-imidazolium-trimethylsilylsulfid ([EMIM]STMS)**

EMIM-methylcarbonat (737 mg, 3.96 mmol, 1.00 eq) wurde in Acetonitril (10 mL) gelöst und die Lösung auf 0 °C gekühlt, $TMS_2S$ (736 mg, 4.13 mmol, 1.04 eq) wurde langsam zugetropft. Die Mischung wurde ein halbe Stunde bei 0 °C gerührt und anschließend das Lösungsmittel im Feinvakuum entfernt. Es blieb ein brauner Honig zurück.
Ausbeute: 86 mg (0,397 mmol, 10%) brauner Honig.
$^1$H-NMR (300.1 MHz, DMSO-$d_6$): δ = -0.07 (s, 9H, *S-TMS),* 1.41 (t, $^3J_{HH}$ = 7.3 Hz, 3H, $CH_2CH_3$), 3.87 (s, 3H, NMe), 4.22 (q, $^3J_{HH}$ = 7.3 Hz , 2H, $CH_2CH_3$), 7.77 (d, $^3J_{HH}$ = 1.9 Hz, 1 H, *H4/5*), 7.87 (d, $^3J_{HH}$ = 1.9 Hz, 1 H, *H4/5*), 9.49 (s, 1 H, *H2*) ppm.
$^{13}$C-NMR (75.5 MHz, DMSO-$d_6$): δ = 8.82 (3C, *S-TMS),* 15.2 (1 C, $CH_2CH_3$), 35.6 (1C, $CH_2CH_3$), 44.0 (1C, N*Me*), 121.9 (1C, *C4/5*), 123.5 (1C, *C4/5*), 136.4 (1C, *C2*) ppm.
$^{29}$Si-NMR (79.5 MHz, DMSO-$d_6$): δ = -0.4 ppm.

**Darstellung von N-Butyl-N-methyl-piperidinium-trimethylsilylsulfid ([BMPip]STMS)**

*N*-Butyl-*N*-methyl-piperidinium-methylcarbonat (1.49 g, 6.45 mmol, 1.00 eq) wurde in Acetonitril (8 mL) gelöst und die Lösung auf 0 °C gekühlt, $TMS_2S$ (1.22 g, 6.81 mmol, 1.06 eq) wurde langsam zugetropft. Die Mischung wurde ein halbe Stunde bei 0 °C und anschließend eine halbe Stunde bei Raumtemperatur gerührt, bevor das Lösungsmittel im Feinvakuum entfernt wurde. Der Rückstand wurde in Diethylether (10 mL) digeriert, die Suspension filtriert und der Rückstand mit Diethylether (5 mL) gewaschen und im Feinvakuum getrocknet.
Ausbeute: 1.48 g (5.66 mmol, 88%).
$^1$H-NMR (300.1 MHz, DMSO-$d_6$): $\delta$ = -0.07 (s, 9H, *S-TMS),* 0.93 (t, $^3J_{HH}$ = 7.3 Hz, 3H, $CH_2CH_3$), 1.25-1.38 (m, 2H, $CH_2CH_3$), 1.48-1.69 (m, 4H), 1.72-1.83 (m, 4H), 3.02 (s, 3H, N*Me*), 3.31-3.41 (m, 6H) ppm.
$^{13}$C-NMR (75.5 MHz, DMSO-$d_6$): $\delta$ = 8.82 (3C, *S-TMS*), 13.5 (1 C), 19.3 (1 C), 20.6 (1 C), 22.9 (1 C), 46.9 (br s, 2C), 59.8 (1 C), 62.1 (br s, 2C) ppm.
$^{29}$Si-NMR (79.5 MHz, DMSO-$d_6$): $\delta$ = -0.4 ppm.

**Darstellung von N-Butyl-N-methyl-piperidinium-trimethylsilylselenid ([BMPip]SeTMS)**

*N*-Butyl-*N*-methyl-piperidinium-methylcarbonat (1.47 g, 6.37 mmol, 1.00 eq) wurde in Acetonitril (8 mL) gelöst und die Lösung auf 0 °C gekühlt, $TMS_2Se$ (1.51 g, 6.70 mmol, 1.05 eq) wurde langsam zugetropft. Die Mischung wurde 40 Minuten bei 0 °C und anschließend eine halbe Stunde bei Raumtemperatur gerührt. Das Lösungsmittel wurde im Feinvakuum entfernt.
Ausbeute: 1.90 g (6.16 mmol, 97%).
$^1$H-NMR (300.1 MHz, DMSO-$d_6$): $\delta$ = -0.08 (s, 9H, Se-*TMS*), 0.93 (t, $^3J_{HH}$ = 7.3 Hz, 3H, $CH_2CH_3$), 1.25-1.37 (m, 2H, $CH_2CH_3$), 1.48-1.69 (m, 4H), 1.72-1.83 (m, 4H), 3.03 (s, 3H, N*Me*), 3.31-3.41 (m, 6H) ppm.
$^{13}$C-NMR (75.5 MHz, DMSO-$d_6$): $\delta$ = 9.14 (3C, *Se-TMS*), 13.5 (1C), 19.2 (1C), 20.6 (1 C), 22.9 (1 C), 46.9 (br s, 2C), 59.8 (1 C), 62.1 (br s, 2C) ppm.
$^{29}$Si-NMR (79.5 MHz, DMSO-$d_6$): $\delta$ = -5.0 ppm.

**Darstellung von N-Butyl-N-methyl-pyrrolidinium-trimethylsilyltellurid ([BMPyr]TeTMS)**

*N*-Butyl-*N*-methyl-pyrrolidinium-methylcarbonat (1.00 g, 4.60 mmol, 1.00 eq) wurde in Acetonitril (8 mL) gelöst und die Lösung auf 0 °C gekühlt, $TMS_2Te$ (1.28 g, 4.66 mmol, 1.01 eq) wurde langsam zugetropft. Die Mischung wurde 30 Minuten bei 0 °C und anschließend eine halbe Stunde bei Raumtemperatur gerührt. Das Lösungsmittel wurde im Feinvakuum entfernt.
Ausbeute: 1.44 g (4.19 mmol, 91 %).
$^1$H-NMR (300.1 MHz, DMSO-$d_6$): $\delta$ = 0.32 (s, 9H, Te-*TMS*), 0.92 (t, $^3J_{HH}$= 7.4 Hz, 3H, $CH_2CH_3$), 1.25-1.38 (m, 2H, $CH_2CH_3$), 1.61-1.75 (m, 4H), 2.08 (br s, 4H), 3.01 (s, 3H, N*Me*), 3.32-3.41 (m, 2H), 3.44-3.56 (m, 4H) ppm.
$^{13}$C-NMR (75.5 MHz, DMSO-$d_6$): $\delta$ = 10.2 (3C, *Te-TMS*), 13.5 (1C), 19.3 (1C), 21.1 (1 C), 25.0 (1 C), 47.6 (br s, 2C), 62.8 (br s, 2C), 63.4 (br s, 1 C) ppm. $^{29}$Si-NMR (79.5 MHz, DMSO-$d_6$): $\delta$ = -27.0 ppm.

**Darstellung von N-Butyl-N-methyl-piperidinium-trimethylsilyltellurid ([BMPip]TeTMS)**

*N*-Butyl-*N*-methyl-piperidinium-methylcarbonat (1.35 g, 5.83 mmol, 1.00 eq) wurde in Acetonitril (8 mL) gelöst und die Lösung auf 0 °C gekühlt, $TMS_2Te$ (1.60 g, 5.84 mmol, 1.00 eq) wurde langsam zugetropft. Die Mischung wurde 30 Minuten bei 0 °C und eine halbe Stunde bei Raumtemperatur gerührt. Das Lösungsmittel wurde im Feinvakuum entfernt.
Ausbeute: 1.78 g (4.98 mmol, 85%).
$^1$H-NMR (300.1 MHz, DMSO-$d_6$): $\delta$ = 0.32 (s, 9H, Te-*TMS*), 0.93 (t, $^3J_{HH}$= 7.1 Hz, 3H, $CH_2CH_3$), 1.26-1.39 (m, 2H, $CH_2CH_3$), 1.48-1.70 (m, 4H), 1.72-1.85 (m, 4H), 3.02 (s, 3H, N*Me*), 3.29-3.42 (m, 6H) ppm.
$^{13}$C-NMR (75.5 MHz, DMSO-$d_6$): $\delta$ = 9.8 (3C, Te-*TMS*), 13.5 (1 C), 19.2 (1 C), 20.6 (1C), 22.9 (1C), 46.9 (br s, 2C), 59.8 (1C), 62.1 (br s, 2C) ppm.$^{29}$Si-NMR (79.5 MHz, DMSO-$d_6$): $\delta$ = -27.2 ppm.

**Darstellung von Methyl-tri-nbutyl-phosphonium-trimethylsilyltellurid ([MeP(nBu)$_3$]TeTMS)**

Methyl-tri-*n*butyl-phosphonium-methylcarbonat (971 mg, 3.32 mmol, 1.00 eq) wurde in Acetonitril (6 mL) gelöst, die Lösung auf 0 °C gekühlt. $TMS_2Te$ (950 mg, 3.47 mmol, 1.04 eq) wurde tropfenweise zugegeben. Die Mischung wurde eine Stunde bei 0 °C und eine halbe Stunde bei Raumtemperatur gerührt und schließlich im Feinvakuum eingeengt.
Ausbeute: 1.29 g (3.09 mmol, 93%).
$^1$H-NMR (300.1 MHz, DMSO-$d_6$): $\delta$ = 0.32 (s, 1H, Te*TMS*), 0.91 (t, $^3J_{HH}$ = 7.0 Hz, 9H, $CH_2CH_3$), 1.32-1.56 (m, 12H,

$CH_2CH_2$), 1.85 (d, $^2J_{HP}$ = 14.0 Hz, 3H, P*Me*), 2.17-2.32 (m, 6H, $PCH_2$) ppm.
$^{13}$C-NMR (75.5 MHz, DMSO-$d_6$): δ = 3.4 (d, $^1J_{CP}$ = 51.3 Hz, 1C, $PCH_3$), 10.1 (s, 3C, Te*TMS*), 13.2 (3C, $CH_2CH_3$), 19.0 (d, $^1J_{CP}$ = 49.1 Hz, 3C, $PCH_2$), 22.6 (d, $J_{CP}$ = 4.3 Hz, 3C, $CH_2$), 23.2 (d, $J_{CP}$ = 15.9 Hz, 3C, $CH_2$) ppm.
$^{31}$P-NMR (101.3 MHz, DMSO-$d_6$): δ = 35.6 ppm.
$^{29}$Si-NMR (79.5 MHz, DMSO-$d_6$): δ = -27.1 (s) ppm.

**Darstellung von Kupfertellurid durch Umsetzung von $CuBr_2$ mit *N*-Butyl-*N*-methyl-pyrrolidinium-trimethylsilyltellurid ([BMPyr]TeTMS)**

$CuBr_2$ (82 mg, 0.37 mmol, 1.0 eq) und [BMPyr]TeTMS (126 mg, 367 μmol, 1.0 eq) wurden in Acetonitril (je 3 mL) gelöst. Während die $CuBr_2$-Lösung zur [BMPyr]TeTMS-Lösung getropft wurde, trat spontane Entfärbung ein und ein dunkler Niederschlag schied sich ab. Niederschlag und Lösung wurden durch Zentrifugieren voneinander getrennt, der Niederschlag mit Acetonitril (5mL) gewaschen. Das bei 25 °C gefällte, elementaranalytisch reine CuTe wurde mittels Röntgenpulverdiffraktometrie untersucht (Fig. 3) und konnte über den Vergleich mit Literaturdaten (Fig. 4, Stand der Technik, Parak et al. J. Am. Chem. Soc. 2013, 135, 7098-7101) als subnanokristallines CuTe identifiziert werden. Im Gegensatz zu dem Literaturbeispiel lassen sich mit den hier beanspruchten Verfahren über die beanspruchten chalkogenidhaltigen ionischen Flüssigkeiten bei niedrigen Temperaturen die reinen binären (und auch multinären) Chalkogenide darstellen. Sie liegen entweder in amorpher (metastabiler) oder subnanokristalliner Fällungsform vor - erkennbar an dem charakteristischen breiten Reflexprofil in Fig. 4 oberste Linie. Die thermodynamisch stabileren nanokristallinen und mikrokristallinen Phasen der Metallchalkogenide lassen sich durch Fällungsreaktionen in den beanspruchten ionischen Flüssigkeiten bei höheren Temperaturen oder durch kontrolliertes Wachstum, der unter kinetisch kontrollierten Bedingungen (tiefen Temperaturen) erhaltenen, amorphen Partikel und Nanokristallite erhalten (z.B. durch einen sogenannten Annealing Prozess).
Fig. 3 zeigt nahezu amorphes CuTe (über [BMPyr]TeTMS hergestellt),
Die zwei Hügel umhüllen die Signalgruppen. Die Partikel sind sehr klein.
Fig. 4 zeigt Nanokristallines CuTe (Stand der Technik),

**Umsetzung von $EuI_2$ mit N-Butyl-N-methyl-piperidinium-trimethylsilylsulfid ([BMPip]STMS)**

$EuI_2$ (96 mg, 0.24 mmol, 1.0 eq) und [BMPip]STMS (62 mg, 0.24 mmol, 1.0 eq) wurden in THF (je 3 mL) gelöst. Die leicht trüben Lösungen wurden über Spritzenfilter filtriert und die [BMPip]STMS-Lösung zu der $EuI_2$-Lösung getropft. Es trat während des Zutropfens eine spontane Reaktion ein, während der sich ein Niederschlag bildete. Die Reaktionsmischung zeigt unter UV-Licht deutliche Fluoreszenzeigenschaften. Lösung und Niederschlag wurden voneinander getrennt, der Niederschlag mit THF (5 mL) gewaschen. Nach Trocknen im Feinvakuum, konnte der Niederschlag mittels $^1$H- und $^{13}$C-NMR-Spektren sowie Elementaranalyse als [BMPip]I identifiziert werden. In Lösung bleibt ein löslicher Europiumsulfid-Vorläufer.

**Synthese von N-Butyl-N-methyl-pyrrolidinium-methylcarbonat**

N-Butyl-pyrrolidin (61.0 mL, 49.9 g, 392 mmol, 1.00 eq) wurde in einem Glasautoklaven mit Dimethylcarbonat (132 mL, 141 g, 1.57 mol, 4.00 eq) und Methanol (25 mL) vereint. Die Mischung wurde 15 Stunden auf 130 °C erwärmt und anschließend auf Raumtemperatur abgekühlt. Die Lösung wurde im Feinvakuum eingeengt, der Rückstand in Acetonitril (50 mL) gelöst und erneut zur Trockene eingedampft. Umkristallisation aus einem Acetonitril-Diethylether-Gemisch liefert die Zielverbindung.
Ausbeute: 60.0 g (276 mmol, 70%).
Die Analytik stimmt mit Literaturdaten überein (J. D. Holbrey et al. Green Chem. 2010, 12, 407-413).

**[Abbildungsbezeichnungen]**

Fig. 1: Kristallstruktur von [EMIM]SH
Fig. 2: Kristallstruktur von [BMPyr]SH
Fig. 3: amorphes CuTe (über [BMPyrip]TeTMS hergestellt)
Fig. 4: Nanokristallines CuTe (Stand der Technik),

**Patentansprüche**

1. Verfahren zur Herstellung einer ionischen Flüssigkeit **dadurch gekennzeichnet, dass** eine ionische Flüssigkeit mit einem Carbonat-Anion, Hydrogencarbonat-Anion oder Organocarbonat-Anion über einen Anionenaustausch mit einer Chalkogenid-Verbindung in das Chalkogenid-Anion überführt wird.

2. Verfahren zur Herstellung einer ionischen Flüssigkeit nach Anspruch 1 **dadurch gekennzeichnet, dass** die Chalkogenid-Verbindung ein Bis(organosilyl)chalkogenid, Wasserstoffchalkogenid, Organosilylhydrogenchalkogenid, Polychalkogenid, N-H-funktionelles Ammoniumchalkogenid und Kombinationen dieser umfasst.

3. Verfahren zur Herstellung einer ionischen Flüssigkeit nach einem der Ansprüche 1 bis 2 **dadurch gekennzeichnet, dass** das Chalkogenid-Anion ein Organosilylchalkogenid, Wasserstoffchalkogenid, Polychalkogenid und Kombinationen dieser umfasst.

4. Verfahren zur Herstellung einer ionischen Flüssigkeit nach einem der Ansprüche 1 bis 3 **dadurch gekennzeichnet, dass** das Chalkogenid-Anion ein zweiwertig negativ geladenes Chalkogenid umfasst.

5. Verfahren zur Herstellung einer ionischen Flüssigkeit nach einem der Ansprüche 1 bis 4 **dadurch gekennzeichnet, dass** als Chalkogen-Komponente Schwefel, Selen, Tellur und Kombinationen dieser eingesetzt werden.

6. Verfahren zur Herstellung einer ionischen Flüssigkeit nach einem der Ansprüche 1 bis 5 **dadurch gekennzeichnet, dass** bei dem Organocarbonat-Anion eine lineare oder verzweigte acyclische oder cyclische Alkylgruppe als Organo-Gruppe an das Carbonat-Anion gebunden ist.

7. Verfahren zur Herstellung einer ionischen Flüssigkeit nach einem der Ansprüche 1 bis 6 **dadurch gekennzeichnet, dass** zur Herstellung des Wasserstoffchalkogenids bevorzugt eine Protonierung des Organocarbonat-Anions mit anschließender Decarboxylierung zu einem Alkohol und zur Herstellung des Organosilyl-chalkogenids eine Desilylierung des Bis(organosilyl)chalkogenids gefolgt von einer Decarboxylierung des intermediär gebildeten O-Silylorganocarbonat-Anions zu einem Organosilylether erfolgt.

8. Verfahren zur Herstellung einer ionischen Flüssigkeit nach einem der Ansprüche 1 bis 7 **dadurch gekennzeichnet, dass** als Edukt ein Kationenprecursor für ionische Flüssigkeiten vorgelegt wird und dieser mit einem Dimethylcarbonat und anschließend in situ mit einer Chalkogenid-Verbindung nach Anspruch 2 umgesetzt wird, so dass eine ionische Flüssigkeit mit Chalkogenid-Anion hergestellt wird.

9. Ionische Flüssigkeit mit einem Kation $Cat^+$ und Anion, wobei die Herstellung auch ein Verfahren nach Anspruch 1 umfasst, **dadurch gekennzeichnet, dass** das Anion ein Organosilyl-Chalkogenid umfasst.

10. Ionische Flüssigkeit nach Anspruch 9 **dadurch gekennzeichnet, dass** das Anion als Chalkogen-Komponente Schwefel, Selen, Tellur und Kombinationen dieser umfasst.

11. Ionische Flüssigkeit nach einem der Ansprüche 9 bis 10 **dadurch gekennzeichnet, dass** die organische Gruppe im Bis(organosilyl)chalkogenid oder im Organosilylchalkogenid bis zu drei unabhängig voneinander lineare oder verzweigte acyclische oder cyclische Alkylgruppen mit einer Kohlenstoffanzahl von 1 bis 20 und oder Wasserstoff umfasst.

12. Ionische Flüssigkeit nach einem der Ansprüche 9 bis 11 **dadurch gekennzeichnet, dass** das Kation $Cat^+$ substituierte Strukturen mit ionischem Stickstoff, Kohlenstoff, Phosphor und / oder Schwefel aufweist.

13. Ionische Flüssigkeit nach einem der Ansprüche 9 bis 12 **dadurch gekennzeichnet, dass** das Kation bevorzugt substituierte Strukturen wie Imidazol, Pyridin, Guanidin, Pyrrolidin, Piperidin, Pyrazol , Verbindungen mit Ammonium-Ion, Phosphonium-Ion, Sulfonium-Ion oder Sulfoxoium-Ion aufweist.

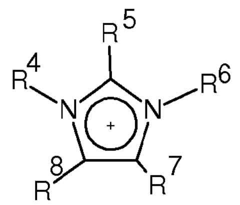 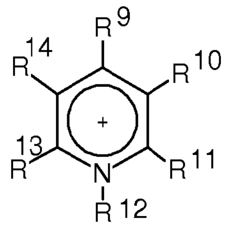 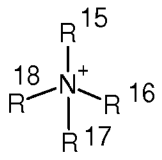 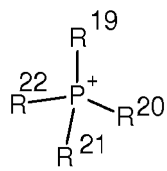 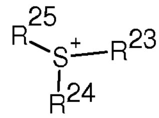

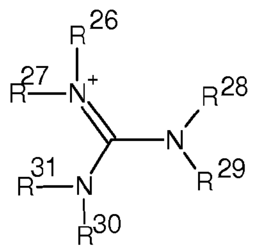 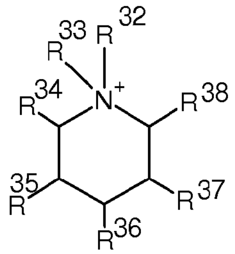 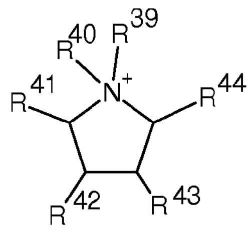 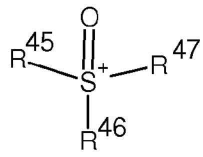

**14.** Verwendung der ionischen Flüssigkeit nach Anspruch 1 und oder 9 unter anderem in folgenden Bereichen:

- als reaktive Komponente in Fällungsreaktionen binärer oder multinärer Metallchalkogenide $M_nM'_mS_xSe_yTe_z$ in organischen Lösungsmitteln oder in der reaktiven ionischen Flüssigkeit im Sinne einer Schmelzflusslonothermalsynthese,
- in der Herstellung von Metallchalkogenid-basierten Dünnfilm-Halbleitermaterialien $M_nM'_mS_xSe_yTe_z$ aus Lösung,
- in der Herstellung von Metallchalkogeniden, Kombinationen dieser oder Dünnfilm-Halbleitermaterialien $M_nM'_mS_xSe_yTe_z$ im Sinne einer Printed Electronics Strategie, wobei die verschiedenen Chalkogenkomponenten in Form verschiedener Lösungen der hier beanspruchten Chalkogenidhaltigen ionischen Flüssigkeiten auf dem Target mit Lösungen reaktiver Metallkomponenten über verschiedene Coating-, Spray- und Print-Verfahren zur Reaktion gebracht werden.

**15.** Verwendung der ionischen Flüssigkeit nach Anspruch 14 unter anderem in folgenden zusätzlichen Bereichen:

- als redoxaktiver Elektrolyt für Batterien, Akkumulatoren und andere elektrochemische Zellen,
- als Redoxmediator in Farbstoffsolarzellen und Quantum Dot sensibilisierten Solarzellen,
- zur Beschichtung von Wafern oder Substraten mit Metall-Chalkogenid-Materialien,
- als Lösungsmittel für chemische Reaktionen,
- als Lösungsmittel und reaktive Komponente für elementare Formen der Chalkogene unter Bildung von ionischen Flüssigkeiten mit Polychalkogenidanionen und Kombinationen dieser.

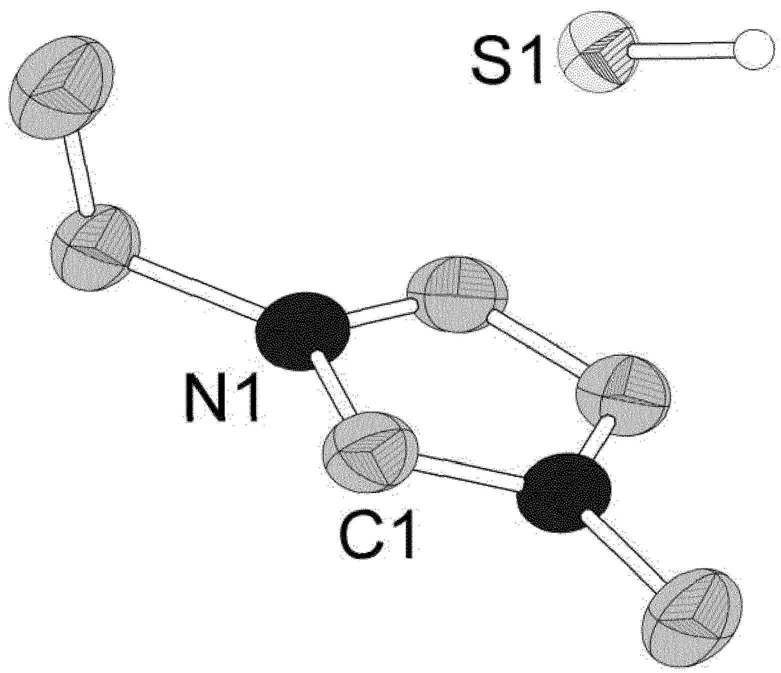

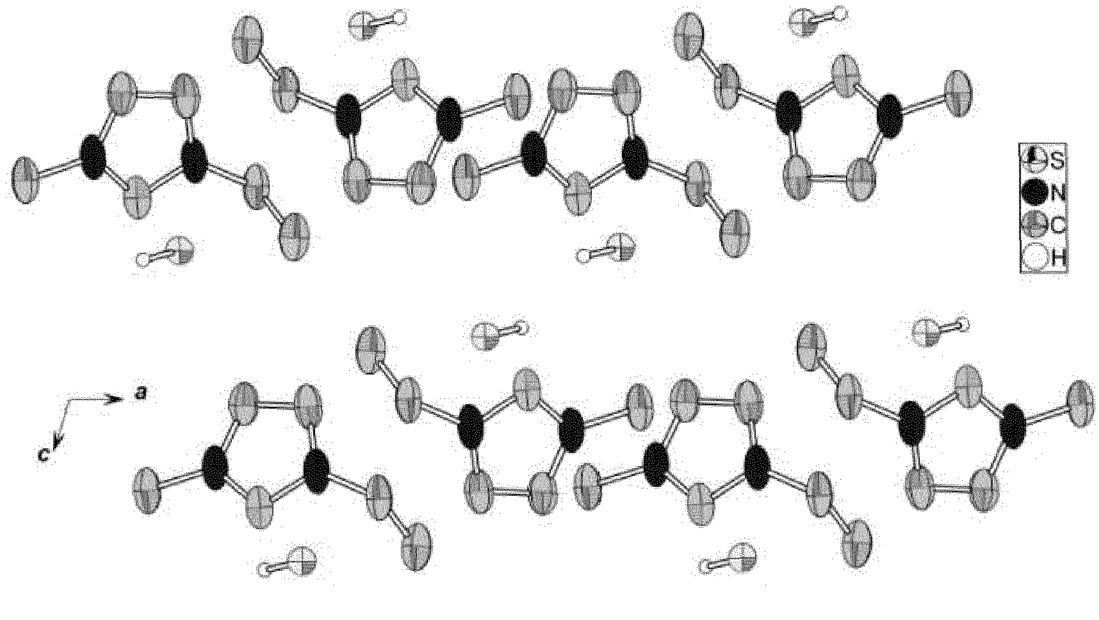

Fig. 1

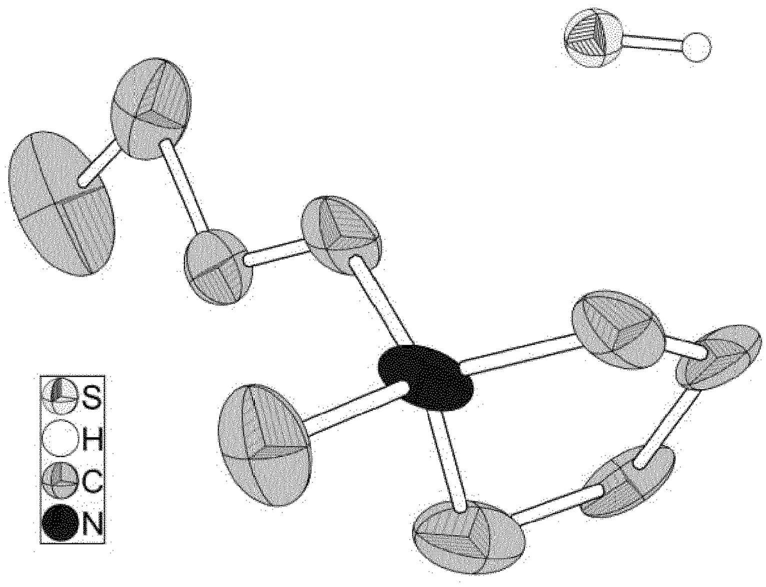


Fig. 2

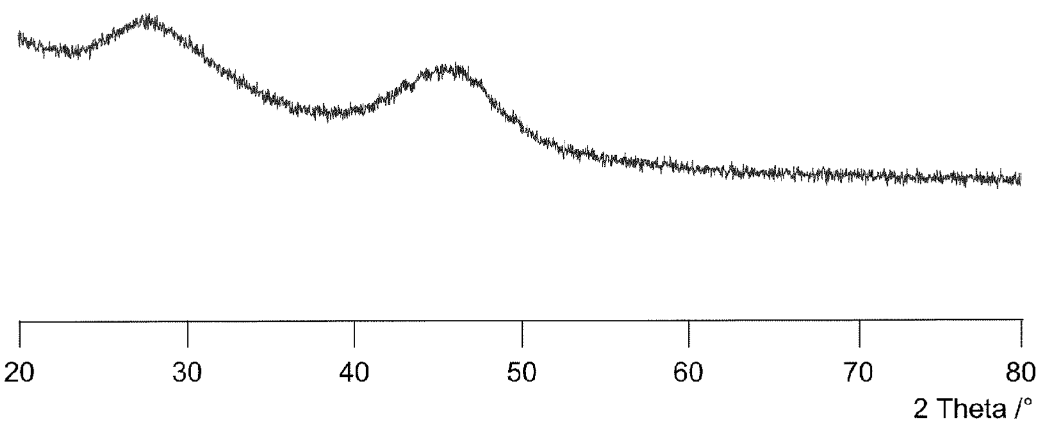
20
30
40
50
60
70
80
2 Theta /°

Fig. 3

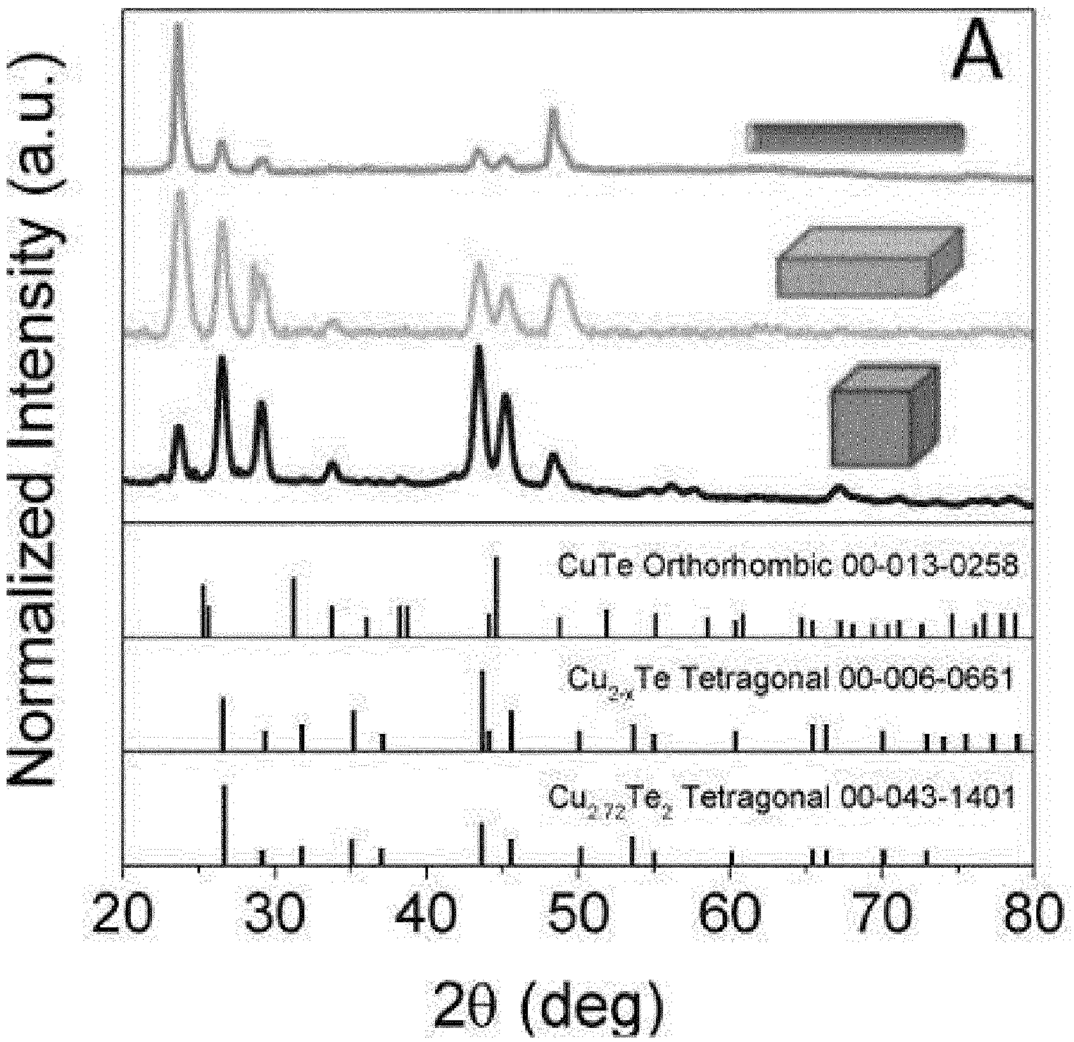
A
Normalized Intensity (a.u.)
CuTe Orthorhombic 00-013-0258
$Cu_{2-x}$Te Tetragonal 00-006-0661
$Cu_{2.72}Te_2$ Tetragonal 00-043-1401
20
30
40
50
60
70
80
2θ (deg)

Fig. 4

Europäisches Patentamt
European Patent Office
Office européen des brevets

# EUROPÄISCHER RECHERCHENBERICHT

Nummer der Anmeldung
EP 13 19 4431

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A,D | EP 2 388 853 A1 (FUNDACION CIDETEC; UNIVERSITAT JAUME I) 23. November 2011 (2011-11-23) * Absatz [0011] - Absatz [0014] * * Absatz [0017] - Absatz [0021] * * Beispiele 1, 2 * * Ansprüche 1-3, 8-10 * ----- | 1-15 | INV. C01B17/00 C07C211/62 C07C211/63 C07C211/64 C07C279/04 C07C321/12 C07C381/00 C07C381/12 C07C391/00 C07C395/00 C07D213/20 C07D231/12 C07D233/56 C07F9/54 H01B1/12 ADD. |
| A,D | CN 102 097 213 B (GUANGZHOU INSTITUTE OF ENERGY CONVERSION) 25. Juli 2012 (2012-07-25) * Zusammenfassung * * Abbildung 1 * ----- | 1-15 | |

RECHERCHIERTE SACHGEBIETE (IPC)

C01B
C07C
C07D
C07F
H01B
H01G
H01L
H01M

Der vorliegende Recherchenbericht wurde für alle Patentansprüche erstellt

1

| Recherchenort | Abschlußdatum der Recherche | Prüfer |
|---|---|---|
| München | 25. April 2014 | Masson, Jean-Pierre |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C03)

Europäisches Patentamt
European Patent Office
Office européen des brevets

# EUROPÄISCHER RECHERCHENBERICHT

Nummer der Anmeldung
EP 13 19 4431

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (IPC) |
| | | | C01B19/00<br>H01G9/20<br>H01L31/042<br>H01M6/16<br>H01M10/0569 |
| | | | RECHERCHIERTE SACHGEBIETE (IPC) |
| | | | |

Der vorliegende Recherchenbericht wurde für alle Patentansprüche erstellt

1

| Recherchenort | Abschlußdatum der Recherche | Prüfer |
|---|---|---|
| München | 25. April 2014 | Masson, Jean-Pierre |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR. EP 13 19 4431

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

25-04-2014

| Im Recherchenbericht angeführtes Patentdokument | Datum der Veröffentlichung | Mitglied(er) der Patentfamilie | Datum der Veröffentlichung |
|---|---|---|---|
| EP 2388853 A1 | 23-11-2011 | EP 2388853 A1<br>EP 2572402 A1<br>WO 2011144697 A1 | 23-11-2011<br>27-03-2013<br>24-11-2011 |
| CN 102097213 B | 25-07-2012 | KEINE | |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2011144697 A
- CN 102097213 B
- WO 9811619 A


### In der Beschreibung aufgeführte Nicht-Patentliteratur


- **PARAK et al.** *J. Am. Chem. Soc.,* 2013, vol. 135, 7098-7101
- **J. D. HOLBREY et al.** *Green Chem.,* 2010, vol. 12, 407-413